# EUROPÄISCHE PATENTANMELDUNG

(11) **EP 0 619 143 A1**
(43) Veröffentlichungstag der Anmeldung: **12.10.1994**
(21) Anmeldenummer: 94102105.7
(22) Anmeldetag: 11.02.1994
(51) Int. Cl.: B01J 23/755, C10G 45/48, C07C 5/10

(54) **Nickel/Aluminiumoxid-Katalysator, Verfahren zu seiner Herstellung, seine Verwendung sowie Verfahren zur Hydrierung von aromatischen Kohlenwasserstoffen mit Hilfe des Katalysators**

(30) Priorität: 03.04.1993 DE 4310971
(71) Anmelder: HÜLS AKTIENGESELLSCHAFT, D-45764 Marl (DE)
(72) Erfinder: Baldauf, Wolfgang, Dr., D-46282 Dorsten (DE); Rupp, Martin, Dr., D-45239 Essen (DE); Bolz, Heinz, D-45894 Gelsenkirchen (DE); Lüken, Hans-Gerd, Dr., D-45772 Marl (DE); Schuler, Joachim, Dr., D-45772 Marl (DE); Nowitzki, Bernd, Dr., D-45772 Marl (DE)

(57) **Zusammenfassung**

Die Erfindung betrifft einen Nickel/Aluminiumoxid-Katalysator mit einem Nickelgehalt von 10 bis 60 Gew.-%, bezogen auf den gesamten Katalysator, wobei die Nickelkristallite einen mittleren Durchmesser von 15 nm bis 50 nm aufweisen.

Den Nickel/Aluminiumoxid-Katalysator stellt man her, indem eine Nickelverbindung mit einer Aluminiumoxidvorläuferverbindung gemischt, die Mischung verformt, der Formkörper getrocknet und der so erhaltene Katalysatorvorläufer in einem wasserstoffhaltigen Gasstrom bei Temperaturen von 230 bis 550 °C aktiviert wird.

Der Nickel-Aluminiumoxid-Katalysator kann in einem Verfahren zur Hydrierung von aromatischen Kohlenwasserstoffen eingesetzt werden.

## Beschreibung

Die Erfindung betrifft einen Nickel/Aluminiumoxid-Katalysator mit einem Nickelgehalt von 10 bis 60 Gew.-%, bezogen auf den gesamten Katalysator, ein Verfahren zu seiner Herstellung sowie seine Verwendung zur Hydrierung von aromatischen Kohlenwasserstoffen.

Außerdem betrifft die Erfindung ein Verfahren zur Hydrierung von aromatischen Kohlenwasserstoffen mit Hilfe des Katalysators.

Mineralölfraktionen werden in vielen Bereichen, wie z. B. in der Lackindustrie, als Lösungsmittel für Lacke verwendet.

Durch Reduzieren des Aromatenanteils in den Mineralölfraktionen lassen sich entaromatisierte Produkte gewinnen, die z. B. in der Lebensmittelindustrie, Pharmazie oder auch in der Kosmetikindustrie, Anwendung finden.

Des weiteren kann auch aromatenreduziertes Kerosin als Düsentreibstoff verwendet werden. Durch die Verminderung des Aromatenanteils ergeben sich Vorteile, z. B. in Form eines verbesserten "smoke point" des Düsentreibstoffs.

Zur Senkung des Aromatengehaltes bieten sich im allgemeinen mehrere Möglichkeiten an, wie sie beispielsweise in der DE-OS 23 05 143 erwähnt werden.

So können z. B. aromatenhaltige Kohlenwasserstoffe durch Behandeln mit Schwefelsäure oder Oleum entaromatisiert werden. Problematisch ist diese Variante allerdings aufgrund des Materialverschleißes durch Korrosion. Weiterhin ist der Umgang mit Schwefelsäure oder Oleum im Hinblick auf die Entsorgung der Abfallschwefelsäure nicht unproblematisch.

Eine andere Methode zur Senkung des Aromatengehaltes ist die Hydrierung der aromatischen Kohlenwasserstoffe mit Hilfe geeigneter Katalysatoren.

So lehrt DE-OS 20 42 166 Katalysatorsysteme zur Hydrierung von aromatischen Kohlenwasserstoffen, wobei Pt oder Rh auf Katalysatorträgern eingesetzt werden.

Allerdings sind solche Edelmetallkatalysatoren aufgrund ihres Anteils an Edelmetall vergleichsweise teuer und beeinflussen dadurch die Wirtschaftlichkeit des Hydrierverfahrens in negativer Weise.

Um Anwendungen der Mineralölfraktionen, z. B. in der Lebensmittelindustrie, zu ermöglichen, muß der Aromatenanteil in den Mineralölfraktionen erheblich gesenkt werden. Will man die Verminderung des Aromatengehaltes durch katalytische Hydrierung erreichen, so sind zur Absenkung des Aromatengehaltes auf ein sehr niedriges Niveau, z. B. in den Konzentrationsbereich von wenigen Gew.-ppm (parts per million) große Katalysatorvolumina erforderlich, was die Wirtschaftlichkeit der katalytischen Hydrierverfahren wesentlich beeinflußt.

DE-OS 23 05 143 offenbart ein Katalysatorsystem auf Basis Nickel und Al₂O₃. Die Herstellung des Katalysators erfolgt über die Fällung von Nickel/Aluminium-Hydroxidcarbonat mit diversen weiteren Verarbeitungsschritten. Unter anderem beinhaltet die Herstellung des Katalysators das Einkneten des gefällten Nickel/Aluminium-Komplexes in Al₂O₃. Dieses Verfahren ist vergleichsweise umständlich.

Der so erhaltene Katalysator kann zur Hydrierung von aromatischen Kohlenwasserstoffen eingesetzt werden. Allerdings verbleiben nach der Hydrierung im Hydrierprodukt noch Restgehalte an Aromaten von ca. 200 Gew.-ppm Außerdem ist die Katalysatorbelastung, ausgedrückt in [g Durchsatz / (cm³ Katalysator · h)], mit ca. 1 g / (cm³ · h) nicht allzu hoch.

In der EP-A 0 290 100 wird ein Katalysator beschrieben, der durch Imprägnierung eines vorgeformten Al₂O₃-Trägers mit einer Nickelsalzlösung hergestellt wird. Aus den beanspruchten Merkmalen lassen sich Nickelkristallitgrößen von ca. 2 bis 8 nm abschätzen. Üblicherweise wird davon ausgegangen, daß Nickelkatalysatoren umso aktiver sind, je kleiner der Kristallitdurchmesser ist. Der Katalysatorträger wird vor der Imprägnierung bei hohen Temperaturen vorbehandelt, um eine bestimmte Modifikation des Al₂O₃ zu erreichen. Durch die Technik der Imprägnierung können nur vergleichsweise geringe Beladungen des Katalysatorträgers mit Nickel erreicht werden. Um den Nickelgehalt des Katalysators auf technisch interessante Werte zu erhöhen, ist es notwendig, die Imprägnierung des Katalysators mehrfach durchzuführen. Dabei muß nach jeder Imprägnierstufe der Katalysator getrocknet bzw. calciniert werden. Durch diese Verfahrensweise ist die Herstellung des Katalysators umständlich, insbesondere dann, wenn hohe Nickelkonzentrationen im Katalysator erreicht werden sollen. Bei Verwendung dieses Katalysators gemäß EP-A 0 290 100 werden Umsätze bei der Hydrierung von aromatischen Kohlenwasserstoffen von 96 % erst bei Temperaturen von ca. 150 °C erreicht. Der Restgehalt an Aromaten im Hydrierprodukt liegt unter den oben aufgeführten Bedingungen bei etwa 7 600 Gew.-ppm. Bei Temperaturen von ca. 175 °C beträgt der Umsatz etwa 99,4 % und die Restkonzentration an Aromaten etwa 1 300 Gew.-ppm.

EP-A 0 092 878 lehrt Katalysatoren mit Nickel auf Al₂O₃, die ebenfalls zur Hydrierung von aromatischen Kohlenwasserstoffen eingesetzt werden. Es werden Nickelkristallitgrößen von ca. 2 bis 8 nm beansprucht. Die Herstellung dieser Katalysatoren erfolgt über eine Ausfällung von Nickelhydroxid auf einem Übergangsaluminiumoxid. Dieses Herstellverfahren ist durch die verschiedenen Teilschritte vergleichsweise umständlich, insbesondere dann, wenn hohe Nickelgehalte auf dem Katalysator erforderlich sind, was z. B. der Fall ist, wenn eine Kontamination des Katalysators durch Katalysatorgifte nicht ausgeschlossen werden kann.

Der Erfindung liegt die Aufgabe zugrunde, einen Nickel/Aluminiumoxid-Katalysator zu entwickeln, der eine große katalytische Aktivität aufweist, eine hohe Katalysatorbelastung ermöglicht und auf einfache Weise hergestellt werden kann. Bei seinem Einsatz zur katalytischen Hydrierung von aromatischen Kohlenwasserstoffen soll ein Restgehalt an aromatischen Kohlenwasserstoffen im Hydrierprodukt von 10 Gew.-ppm und kleiner erzielt werden.

Es wurde nun überraschenderweise gefunden, daß ein Nickel/Aluminiumoxid-Katalysator mit einem Nickelgehalt von 10 bis 60 Gew.-% und einem mittleren Durchmesser der Nickelkristallite von 15 nm bis 50 nm eine sehr hohe Katalysatorbelastung ermöglicht sowie eine hervorragende katalytische Aktivität zeigt. Es lassen sich mit seiner Hilfe bei der katalytischen Hydrierung von aromatischen Kohlenwasserstoffen zu den entsprechenden cycloaliphatischen Verbindungen ohne weiteres Restgehalte an aromatischen Kohlenwasserstoffen im Hydrierprodukt von unter 10 Gew.-ppm erreichen. Außerdem läßt er sich auf besonders einfache weise herstellen, indem man eine Nickelverbindung mit einer Aluminiumoxid-Vorläuferverbindung vermischt, die Mischung verformt, den Formkörper trocknet und den so erhaltenen Katalysatorvorläufer in einem wasserstoffhaltigen Gasstrom bei Temperaturen von 230 bis 550 °C aktiviert.

Gegenstand der vorliegenden Erfindung ist daher ein Nickel/Aluminiumoxid-Katalysator mit einem Nickelgehalt von 10 bis 60 Gew.-%, bezogen auf den gesamten Katalysator, der dadurch gekennzeichnet ist, daß die Nickelkristallite einen mittleren Durchmesser von 15 nm bis 50 nm aufweisen.

Des weiteren ist Gegenstand der vorliegenden Erfindung ein Verfahren zur Herstellung des Nickel/Aluminiumoxid-Katalysators nach den Ansprüchen 1 bis 6, das dadurch gekennzeichnet ist, daß eine Nickelverbindung mit einer Aluminiumoxid-Vorläuferverbindung gemischt, die Mischung verformt, der Formkörper getrocknet und der so erhaltene Katalysatorvorläufer in einem wasserstoffhaltigen Gasstrom bei Temperaturen von 230 bis 550 °C aktiviert wird.

Darüber hinaus ist Gegenstand der vorliegenden Erfindung die Verwendung des Nickel/Aluminiumoxid-Katalysators nach den Ansprüchen 1 bis 6 zur Hydrierung von aromatischen Kohlenwasserstoffen.

Außerdem ist Gegenstand der vorliegenden Erfindung ein Verfahren zur Hydrierung von aromatischen Kohlenwasserstoffen, das dadurch gekennzeichnet ist, daß ein Nickel/Aluminiumoxid-Katalysator nach den Ansprüchen 1 bis 6 eingesetzt wird.

Vorzugsweise wird beim erfindungsgemäßen Verfahren zur Herstellung des erfindungsgemäßen Katalysators ein basisches Nickelcarbonat mit einer Aluminiumoxid-Vorläuferverbindung gemischt, die Mischung verformt, der Formkörper getrocknet und der so erhaltene Katalysatorvorläufer in einem wasserstoffhaltigen Gasstrom bei Temperaturen von 230 bis 550 °C aktiviert.

Zur Verbesserung der Mischbarkeit bzw. Verformbarkeit der Masse kann die Aluminiumoxidvorläuferverbindung vor Einmischen der Nickelverbindung mit sogenannten Peptisierungshilfsmitteln, wie z. B. einer Säure oder Base, vorbehandelt werden. Weiterin kann der Feuchtigkeitsgehalt der Mischung durch Zusatz oder Entzug von Wasser variiert werden.

Ebenfalls unter dem Aspekt der Verbesserung der Mischbarkeit bzw. Verformbarkeit können in die Mischansätze übliche Gleitmittel, wie beispielsweise Graphit, Cellulose, Cellulosederivate etc., eingebracht werden. Die so erhaltene Mischung wird in an sich bekannter Weise verformt. Als Formkörper lassen sich u. a. Tabletten oder auch Extrudate, jeweils in verschiedener Gestalt, wie z. B. Vollzylinder, Ringe, polylobäre Strukturen etc., und Abmessung, herstellen. Je nach Gestalt des Formkörpers kann der Feuchtegehalt der Mischung zwecks besserer Verformbarkeit variiert werden.

Gegebenenfalls kann der Katalysatorvorläufer nach der Verformung und Trocknung und vor seiner Aktivierung noch einer Calcinierung, z. B. in Luft oder einem Inertgas, wie beispielsweise Stickstoff, unterzogen werden. Nach der Aktivierung des Katalysatorvorläufers in einem wasserstoffhaltigen Gasstrom kann der Katalysator zum Zwecke der besseren Handhabung noch passiviert werden. Die Passivierung des Katalysators kann beispielsweise in einem Inertgasstrom, der noch einen geringen Anteil an einem oxidierenden Gas, wie z. B. Sauerstoff, enthält, erfolgen. Als Inertgas kann z. B. Stickstoff verwendet werden.

Der bei der Aktivierung erhaltene Nickel-Reduktionsgrad des Katalysators, ausgedrückt als prozentualer Anteil metallischen Nickels am Gesamtnickelgehalt des Katalysators, liegt geeigneterweise im Bereich zwischen 35 und 70 %.

Durch den der Aktivierung des Katalysatorvorläufers ggf. vorangehenden Verfahrensschritt der Calcinierung ändern sich die katalytischen Eigenschaften des Katalysators nicht. So kann beispielsweise die BET-Oberfläche des Katalysators unabhängig von einem der Aktivierung vorangehenden Verfahrensschritt der Calcinierung im Bereich von 100 bis 250 m²/g Katalysator liegen.

Der erfindungsgemäße Katalysator weist vorzugsweise ein Porenvolumen von 0,3 bis 0,75 cm³/g Katalysator auf. Vorzugsweise entfallen beim erfindungsgemäßen Katalysator 15 bis 75 % des Porenvolumens auf Poren mit einem Durchmesser von > 100 nm, besonders vorzugsweise weist der erfindungsgemäße Katalysator die folgende Porenvolumenverteilung auf:

| Porendurchmesser (nm) | Anteil des Porenvolumens (%) |
|---|---|
| ≦ 2 | 5 - 15 |
| ≦ 50 | 20 - 70 |
| ≦ 100 | 25 - 85 |
| > 100 | 15 - 75 |

Der erfindungsgemäße Katalysator kann γ-Aluminiumoxid und/oder η-Aluminiumoxid und/oder Boehmit enthalten.

Vorzugsweise weist der Frischkatalysator das folgende Röntgenbeugungsmuster auf:

| Reflex 2 ϑ [Grad], Strahlung Cukα̂ | Reflexbreite* | relative Intensität [I/I₀] |
|---|---|---|
| 14,49 (± 0,4) | b | 0 - 15 |
| 19,4 (± 1,2) | sb | 0 - 10 |
| 26,72 (± 0,1) | ss | 0 - 30 |
| 28,44 (± 0,4) | b | 0 - 15 |
| 37,68 (± 0,4) | b bis sb | 5 - 20 |
| 44,75 (± 0,2) | s bis b | 100 |
| 49,17 (± 0,4) | b bis sb | 0 - 15 |
| 52,02 (± 0,3) | s bis b | 20 - 40 |
| 54,78 (± 0,4) | s bis b | 0 - 10 |
| 63,16 (± 1,0) | sb | 5 - 20 |
| 65,80 (± 1,0) | sb | 5 - 15 |
| 72,14 (± 0,4) | b bis sb | 0 - 10 |
| 76,64 (± 0,3) | s bis b | 10 - 30 |

| | | |
|---|---|---|
| * b = breit sb = sehr breit ss = sehr schmal s = schmal | | |

Als Frischkatalysator wird der erfindungsgemäße Katalysator bezeichnet, der nach der Aktivierung des Katalysatorvorläufers im wasserstoffhaltigen Gasstrom und anschließender Passivierung vorliegt.

Das erfindungsgemäße Verfahren zur Hydrierung von aromatischen Kohlenwasserstoffen wird vorzugsweise bei Drücken von 5 bis 250 bar abs. durchgeführt, und es wird vorzugsweise bei Temperaturen von 80 bis 300 °C durchgeführt.

Geeigneterweise kann man beim erfindungsgemäßen Hydrierverfahren ein Molverhältnis von Wasserstoff zu Aromaten von 3 bis 500 einstellen.

U. a. eignen sich als Einsatzstoffe im erfindungsgemäßen Hydrierverfahren aromatenhaltige Mineralölfraktionen, die Siedebereiche von 20 bis 350 °C aufweisen. Es können also aromatenhaltige Mineralölfraktionen mit Siedebereichen von 20 bis 350 °C zur Hydrierung eingesetzt werden.

Der Aromatengehalt der im erfindungsgemäßen Hydrierverfahren einsetzbaren Mineralölfraktionen ist nicht begrenzt, da, wie nachstehend erläutert, durch geeignete Maßnahmen eine Überhitzung des erfindungsgemäßen Katalysators durch die freiwerdende Reaktionswärme ausgeschlossen wird.

Der erfindungsgemäße Katalysator kann vorteilhafterweise innerhalb des Reaktors in separate Katalysatorbetten unterteilt werden. An geeigneten Stellen zwischen den Katalysatorbetten können Einrichtungen vorgesehen werden, mit deren Hilfe man Kaltgas, beispielsweise in Form von Wasserstoff, einbringen kann, um das aus dem der Kaltgaseinbringung vorangehenden Katalysatorbett austretende heiße Reaktionsprodukt abzukühlen. Unter Kaltgas wird ein Gas verstanden, dessen Temperatur erheblich unter der Temperatur der Katalysatorbetten liegt. Vorzugsweise wird als Kaltgas Wasserstoff oder ein wasserstoffhaltiges Gasgemisch eingesetzt. Man kann also vorteilhafterweise den erfindungsgemäßen Katalysator im Reaktor in separaten Katalysatorbetten anordnen und zwischen den Betten Kaltgas einbringen. Es läßt sich auf diese Weise eine Überhitzung des erfindungsgemäßen Katalysators durch die freiwerdende Reaktionswärme bei weitergehender Hydrierung im nachfolgenden Katalysatorbett vermeiden.

Gleichzeitig läßt es sich durch eine vergleichsweise geringere, wärmeren Wasserstoff enthaltende Gasmenge am Eintritt des ersten Katalysatorbettes ermöglichen, den erfindungsgemäßen Katalysator durch die freiwerdende Reaktionswärme schneller aufzuheizen. Somit kann eine möglichst vollständige Ausnutzung des gesamten Katalysatorbettes erreicht werden. Der zur Hydrierung der aromatischen Kohlenwasserstoffe verbrauchte Wasserstoff kann durch eine Wasserstoff enthaltende Gasmenge so ergänzt werden, daß das Molverhältnis von Wasserstoff zu Aromaten konstant bleibt.

Aufgrund einer Anreicherung von Verunreinigungen im wasserstoffhaltigen Gasstrom, z. B. durch Inertgase wie Stickstoff, kann es notwendig sein, einen Teil des Gasstromes auszuschleusen und durch einen Gasstrom mit einem höheren Wasserstoffgehalt zu ersetzen.

Ein unverbrauchten Wasserstoff enthaltender Gasstrom kann rezirkuliert werden. Der Anteil an eingebrachtem Kaltgas, der zur Abführung der Reaktionswärme dient, hängt von der Konzentration der Aromaten im Einsatzstoff ab.

Der Schwefel- und Stickstoffgehalt im Einsatzstoff für das erfindungsgemäße Hydrierverfahren sollte vorzugsweise weniger als 2 Gew.-ppm betragen.

Mit Hilfe des erfindungsgemäßen Verfahrens zur Hydrierung von aromatischen Kohlenwasserstoffen lassen sich ohne weiteres Restaromatengehalte im Hydrierprodukt von kleiner als 10 Gew.-ppm bei gleichzeitig hoher Katalysatorbelastung, vergleichsweise niedrigen Hydriertemperaturen und hohen Umsätzen an Aromaten erreichen.

Die Erfindung wird durch die folgenden Beispiele näher erläutert:

### Beispiel 1

4 000 g Aluminiumoxidhydroxidhydrat werden als Aluminiumoxidvorläuferverbindung in einem Mischer mit wäßriger Salpetersäure so befeuchtet, daß die Zubereitung einen Feuchtegehalt, ermittelt durch 16stündiges Trocknen bei 120 °C, von 40 % aufweist. Der Nitratgehalt im getrockneten Produkt beträgt 3 Gew.-%.

Der Zubereitung werden 253 g Graphit und 12 116 g Nickelhydroxidcarbonat zugegeben und alle Komponenten miteinander innig vermischt. Es wird ein Feuchtegehalt der Mischung von 40 % eingestellt.

Aus der Mischung werden Extrudate in Form von Vollzylindern hergestellt. Nach der Verformung werden die Formkörper bei einer Temperatur von 110 °C für 16 Stunden getrocknet.

Die Aktivierung des Katalysatorvorläufers erfolgt bei einer Temperatur von 400 °C in einem Gemisch aus Stickstoff als Inertgas und Wasserstoff, wobei das Mischungsverhältnis von Inertgas zu Wasserstoff 1 zu 1 beträgt.

Nach erfolgter Aktivierung wird der Katalysator zur Passivierung zwecks besserer Handhabung bei einer Temperatur von ca. 30 °C mit Luft als Passivierungsmittel behandelt.

Der so hergestellte Frischkatalysator weist die folgenden Merkmale auf:

| | |
|---|---|
| Nickel-Gehalt | 50,3 Gew.-% |
| Nickel-Kristallitgröße | 25 nm |
| BET-Oberfläche | 180 m²/g |
| Porenvolumen | 0,51 cm³/g |

Verteilung des Porenvolumens:

| Porendurchmesser (nm) | Anteil des Porenvolumens (%) |
|---|---|
| ≦ 100 | 56 |
| > 100 | 44 |

| Röntgenbeugungsmuster: | | |
|---|---|---|
| Reflex 2 ϑ [Grad], Strahlung CuKα̂ | Reflexbreite* | relative Intensität [I/I₀] |
| 14,49 (± 0,4) | b | 10 |
| 26,72 (± 0,1) | ss | 26 |
| 28,44 (± 0,4) | b | 10 |
| 37,68 (± 0,4) | b | 16 |
| 44,75 (± 0,2) | s bis b | 100 |
| 49,17 (± 0,4) | b bis sb | 11 |
| 52,02 (± 0,3) | s bis b | 31 |
| 54,78 (± 0,4) | b | 7 |
| 63,16 (± 1,0) | sb | 12 |
| 65,80 (± 1,0) | sb | 10 |
| 72,14 (± 0,4) | b bis sb | 6 |
| 76,64 (± 0,3) | s bis b | 20 |

| | | |
|---|---|---|
| * s = schmal ss = sehr schmal b = breit sb = sehr breit | | |

### Beispiel 2

In analoger Weise zu Beispiel 1 wird ein Katalysator hergestellt, wobei man den Feuchtegehalt der Zubereitung auf einen Wert von 37 % einstellt.

Nach der Trocknung wird der Katalysatorvorläufer einer thermischen Behandlung in Luft bei einer Temperatur von 300 °C unterzogen.

Die sich nach der thermischen Behandlung anschließende Aktivierung des Katalysatorvorläufers erfolgt wie in Beispiel 1, allerdings bei einer Temperatur von 350 °C. Nach der Passivierung wie in Beispiel 1 wird ein Frischkatalysator mit den folgenden Merkmalen erhalten:

| | |
|---|---|
| Nickel-Gehalt | 48,0 Gew.-% |
| Nickel-Kristallitgröße | 32 nm |
| BET-Oberfläche | 160 m²/g |
| Porenvolumen | 0,59 cm³/g |

### Verteilung des Porenvolumens:

| Porendurchmesser (nm) | Anteil des Porenvolumens (%) |
|---|---|
| ≦ 100 | 59 |
| > 100 | 41 |

| Röntgenbeugungsmuster: | | |
|---|---|---|
| Reflex 2 ϑ [Grad], Strahlung CuKα̂ | Reflexbreite* | relative Intensität [I/I₀] |
| 19,4 (± 1,2) | sb | 2 |
| 26,8 (± 0,1) | ss | 17 |
| 37,63 (± 0,4) | b bis sb | 9 |
| 44,76 (± 0,2) | s bis b | 100 |
| 52,11 (± 0,3) | s bis b | 27 |
| 54,90 (± 0,2) | s | 5 |
| 63,80 (± 0,6) | sb | 7 |
| 66,66 (± 0,4) | b | 10 |
| 76,73 (± 0,3) | s bis b | 21 |

| | | |
|---|---|---|
| * s = schmal ss = sehr schmal b = breit sb = sehr breit | | |

### Beispiel 3

Ein Frischkatalysator gemäß Beispiel 1 wird in einer Flüssigphasenkreislaufapparatur, bestehend aus Reaktor, Wärmetauscher, Abscheider und Pumpe, vorgelegt.
Nach Reaktivierung des Frischkatalysators mit Wasserstoff bei einer Temperatur von ca. 150 °C wird die Apparatur mit 1 000 cm³ an C₁₀-C₂₄-Paraffinen als Einsatzstoff befüllt. Der Gehalt an Aromaten im Einsatzstoff beträgt 3 Gew.-%, der Siedebereich des Einsatzstoffes erstreckt sich von einer Temperatur von 190 bis 275 °C. Es wird bei einem Druck von 24 bar abs., einer Temperatur von 140 °C und einem Molverhältnis von Wasserstoff zu Aromaten von 223 hydriert.

Nach einer Verweilzeit von 0,5 h beträgt der Restaromatengehalt im Hydrierprodukt 9 Gew.-ppm.

Der Umsatz an Aromaten beläuft sich auf 99,97 %, und die Katalysatorbelastung liegt bei 2 g/cm³ · h.

### Beispiel 4

57 g eines Frischkatalysators gemäß Beispiel 1 werden in einen Integralreaktor eingefüllt und bei einer Temperatur von 150 °C mit Wasserstoff reaktiviert. Anschließend wird ein Schwerbenzinschnitt mit einem Gehalt an Aromaten von 3 Gew.-% und einem Siedebereich von 52 °C bis 286 °C über den Katalysator geleitet und hydriert. Die Katalysatorbelastung beträgt 3 g Benzin / (g Katalysator · h). Als Molverhältnis von Wasserstoff zu Aromaten wird ein Wert von 160 eingestellt. Die Reaktionstemperatur beträgt 150 °C und der Druck 30 bar abs.

Es verbleibt im Hydrierprodukt ein Restgehalt an Aromaten von 8 Gew.-ppm. Der Umsatz an Aromaten beträgt 99,97 %.

### Beispiel 5

Ein Frischkatalysator gemäß Beispiel 1 wird in einer Versuchsanlage mit zwei Reaktoren und zwei Katalysatorbetten je Reaktor eingesetzt. Nach Reaktivierung im Wasserstoffstrom bei einer Temperatur von 150 °C wird Kerosin mit einem Gehalt an Aromaten von 22,7 Gew.-% und einem Siedebereich von 116 °C bis 287 °C kontinuierlich bei einem Druck von 150 bar abs. und einer Katalysatorbelastung von 4 g Kerosin / (g Katalysator · h) hydriert.

Das Molverhältnis von Wasserstoff zu Aromaten beträgt etwa 27. Hinter jedem Katalysatorbett ist die Möglichkeit gegeben, durch Kaltgaszusatz, in diesem Fall Wasserstoff, die Katalysatortemperatur zu steuern.

Die Reaktoreingangstemperatur des ersten Katalysatorbettes beträgt 115 °C. Zur Steuerung der Reaktionstemperatur wird zwischen den Katalysatorbetten soviel Wasserstoff als Kaltgas zugesetzt, daß die Reaktionstemperatur in den nachfolgenden Katalysatorbetten maximal auf 190 °C ansteigt. Es wird ein Restgehalt an Aromaten im Hydrierprodukt von 2 Gew.-ppm gefunden.

Der Umsatz an Aromaten beträgt > 99,999 %.

### Beispiel 6

Beispiel 5 wird mit einem Frischkatalysator gemäß Beispiel 1 wiederholt. Als Einsatzstoff wird aber ein Schwerbenzinschnitt gemäß Beispiel 4 verwendet, und es wird kein Kaltgas zugesetzt. Ein Zusatz von Kaltgas ist nicht erforderlich, da durch die Konzentration an Aromaten im Einsatzstoff von 3 Gew.-% keine übermäßige Erwärmung des Katalysators zu erwarten ist. Die Reaktoreingangstemperatur beträgt 130 °C und die maximale Reaktionstemperatur 165 °C.

Das Hydrierprodukt enthält nur noch 1 Gew.-ppm an Aromaten. Der Umsatz an Aromaten beträgt > 99,99 %.

### Beispiel 7

Beispiel 5 wird mit einem Mittelbenzinschnitt mit einem Siedebereich von 50 - 165 °C als Einsatzstoff wiederholt. Der Aromatengehalt im Einsatzstoff beträgt 3 Gew.-%. Ein Zusatz von Kaltgas ist aus dem in Beispiel 6 genannten Grund nicht notwendig.

Das Hydrierprodukt weist einen Restgehalt an Aromaten von 1 Gew.-ppm auf, und der Umsatz an Aromaten beträgt > 99,99 %.

## Patentansprüche

1. Nickel/Aluminiumoxid-Katalysator mit einem Nickelgehalt von 10 bis 60 Gew.-%, bezogen auf den gesamten Katalysator,
dadurch gekennzeichnet,
daß die Nickelkristallite einen mittleren Durchmesser von 15 nm bis 50 nm aufweisen.

2. Nickel/Aluminiumoxid-Katalysator nach Anspruch 1,
dadurch gekennzeichnet,
daß der Katalysator ein Porenvolumen von 0,3 bis 0,75 cm³/g Katalysator aufweist.

3. Nickel/Aluminiumoxid-Katalysator nach Anspruch 2,
dadurch gekennzeichnet,
daß 15 bis 75 % des Porenvolumens auf Poren mit einem Durchmesser von > 100 nm entfallen.

4. Nickel/Aluminiumoxid-Katalysator nach Anspruch 3,
dadurch gekennzeichnet,
daß der Katalysator die folgende Porenvolumenverteilung aufweist:
| Porendurchmesser (nm) | Anteil des Porenvolumens (%) |
|---|---|
| ≦ 2 | 5 - 15 |
| ≦ 50 | 20 - 70 |
| ≦ 100 | 25 - 85 |
| > 100 | 15 - 75 |

5. Nickel/Aluminiumoxid-Katalysator nach den Ansprüchen 1 bis 4,
dadurch gekennzeichnet,
daß der Katalysator γ-Aluminiumoxid und/oder η-Aluminiumoxid und/oder Boehmit enthält.

6. Nickel/Aluminiumoxid-Katalysator nach Anspruch 5,
dadurch gekennzeichnet,
daß der Frischkatalysator das folgende Röntgenbeugungsmuster aufweist:
| Reflex 2 ϑ [Grad], Strahlung CuKα̂ | Reflexbreite* | relative Intensität [I/I₀] |
|---|---|---|
| 14,49 (± 0,4) | b | 0 - 15 |
| 19,4 (± 1,2) | sb | 0 - 10 |
| 26,72 (± 0,1) | ss | 0 - 30 |
| 28,44 (± 0,4) | b | 0 - 15 |
| 37,68 (± 0,4) | b bis sb | 5 - 20 |
| 44,75 (± 0,2) | s bis b | 100 |
| 49,17 (± 0,4) | b bis sb | 0 - 15 |
| 52,02 (± 0,3) | s bis b | 20 - 40 |
| 54,78 (± 0,4) | s bis b | 0 - 10 |
| 63,16 (± 1,0) | sb | 5 - 20 |
| 65,80 (± 1,0) | sb | 5 - 15 |
| 72,14 (± 0,4) | b bis sb | 0 - 10 |
| 76,64 (± 0,3) | s bis b | 10 - 30 |
| | | |
|---|---|---|
| * b = breit sb = sehr breit ss = sehr schmal s = schmal | | |

7. Verfahren zur Herstellung des Nickel/Aluminiumoxid-Katalysators nach den Ansprüchen 1 bis 6,
dadurch gekennzeichnet,
daß eine Nickelverbindung mit einer Aluminiumoxidvorläuferverbindung gemischt, die Mischung verformt, der Formkörper getrocknet und der so erhaltene Katalysatorvorläufer in einem wasserstoffhaltigen Gasstrom bei Temperaturen von 230 bis 550 °C aktiviert wird.

8. Verfahren nach Anspruch 7,
dadurch gekennzeichnet,
daß ein basisches Nickelcarbonat mit einer Aluminiumoxidvorläuferverbindung gemischt, die Mischung verformt, der Formkörper getrocknet und der so erhaltene Katalysatorvorläufer in einem wasserstoffhaltigen Gasstrom bei Temperaturen von 230 bis 550 °C aktiviert wird.

9. Verfahren zur Hydrierung von aromatischen Kohlenwasserstoffen,
dadurch gekennzeichnet,
daß ein Nickel/Aluminiumoxid-Katalysator nach den Ansprüchen 1 bis 6 eingesetzt wird.

10. Verfahren nach Anspruch 9,
dadurch gekennzeichnet,
daß die Hydrierung bei Drücken von 5 bis 250 bar abs. durchgeführt wird.

11. Verfahren nach den Ansprüchen 9 und 10,
dadurch gekennzeichnet,
daß die Hydrierung bei Temperaturen von 80 bis 300 °C durchgeführt wird.

12. Verfahren nach den Ansprüchen 9 bis 11,
dadurch gekennzeichnet,
daß ein Molverhältnis von Wasserstoff zu Aromaten von 3 bis 500 eingestellt wird.

13. Verfahren nach den Ansprüchen 9 bis 12,
dadurch gekennzeichnet,
daß aromatenhaltige Mineralölfraktionen mit Siedebereichen von 20 bis 350 °C zur Hydrierung eingesetzt werden.

14. Verfahren nach den Ansprüchen 9 bis 13,
dadurch gekennzeichnet,
daß der Katalysator im Reaktor in separaten Katalysatorbetten angeordnet und zwischen den Betten Kaltgas eingebracht wird.

15. Verwendung des Nickel/Aluminiumoxid-Katalysators nach den Ansprüchen 1 bis 6 zur Hydrierung von aromatischen Kohlenwasserstoffen.
